# EUROPEAN PATENT APPLICATION

(11) **EP 2 186 891 A1**
(43) Date of publication of application: **19.05.2010**
(21) Application number: 08792113.6
(22) Date of filing: 01.08.2008
(51) Int. Cl.: C12N 15/00, C12N 1/19, C12N 15/09, C12P 7/06, C12P 7/56, C12R 1/865

(54) **YEAST FOR TRANSFORMATION, TRANSFORMATION METHOD AND METHOD OF PRODUCING SUBSTANCE**

(30) Priority: 01.08.2007 JP 2007200978
(71) Applicant: Toyota Jidosha Kabushiki Kaisha, Toyota-shi, Aichi 471-8571 (JP)
(72) Inventor: SAITO, Satoshi, Toyota-shi Aichi 471-8571 (JP); TAKAHASHI, Kazushi, Toyota-shi Aichi 471-8571 (JP); MIYATA, Kayo, Toyota-shi Aichi 471-8571 (JP); KONDO, Akihiko, Kobe-shi Hyogo 657-8501 (JP)
(74) Representative: Mallalieu, Catherine Louise
(86) International application number: PCT/JP2008/063900
(87) International publication number: WO 2009/017233

(57) **Abstract**

Yeast for transformation is provided, which enables introduction of a greater number of copies of a target gene or a greater number of types of target genes. A method provided herein comprises the steps of introducing a target gene into yeast for transformation, which has homothallic properties and a plurality of selection markers, and selecting a strain in which the target gene has been introduced based on the selection markers in the yeast for transformation, whereby multiple target genes were introduced owing to the homothallic properties of the yeast for transformation.

SEQUENCE LISTING

<110> Toyota Jidosha Kabushiki Kaisha

<120> Yeast for transformation, transformation method and method of
producing substance

<130> P039487EP

<140> 08792113.6
<141> 2008-08-01

<150> JP 2007-200978
<151> 2007-08-01

<160> 8

<170> PatentIn version 3.4

<210> 1
<211> 20
<212> DNA
<213> Artificial

<220>
<223> Synthetic DNA

<400> 1
tccactccga ccacatgaag 20

<210> 2
<211> 20
<212> DNA
<213> Artificial

<220>
<223> Synthetic DNA

<400> 2
tggggtagaa gctgggacag 20

<210> 3
<211> 21
<212> DNA
<213> Artificial

<220>
<223> Synthetic DNA

<400> 3
ttctctaaag atcgatgtcg c 21

<210> 4
<211> 22
<212> DNA
<213> Artificial

<220>
<223> Synthetic DNA

<400> 4
gagacaatct tcttgtctgg ag 22

<210> 5
<211> 23
<212> DNA
<213> Artificial

<220>
<223> Synthetic DNA

<400> 5
gttttcaagg aattagacac tgc 23


<210> 6
<211> 23
<212> DNA
<213> Artificial

<220>
<223> Synthetic DNA

<400> 6
caacagtctt ttgagtagca gtc 23


<210> 7
<211> 40
<212> DNA
<213> Artificial

<220>
<223> Synthetic DNA

<400> 7
gatctccatg gctgatgaac tggcgttctc tcctcctttc 40


<210> 8
<211> 40
<212> DNA
<213> Artificial

<220>
<223> Synthetic DNA

<400> 8
tggcgctcga gccttgcacc ttcgggagcg ccgcgtgaag 40

SEQUENCE LISTING

<110>  Toyota Jidosha Kabushiki Kaisha

<120>  Yeast for transformation, a method of transformation and a method
       for producing a product.

<130>  PH-3643-PCT

<150>  JP 2007-200978
<151>  2007-08-01

<160>  8

<170>  PatentIn version 3.4

<210>  1
<211>  20
<212>  DNA
<213>  Artificial

<220>
<223>  Synthetic DNA

<400>  1
tccactccga ccacatgaag                                                   20


<210>  2
<211>  20
<212>  DNA
<213>  Artificial

<220>
<223>  Synthetic DNA

<400>  2
tggggtagaa gctgggacag                                                   20


<210>  3
<211>  21
<212>  DNA
<213>  Artificial

<220>
<223>  Synthetic DNA

<400>  3
ttctctaaag atcgatgtcg c                                                 21


<210>  4
<211>  22
<212>  DNA
<213>  Artificial

<220>
<223>  Synthetic DNA

<400>  4

gagacaatct tcttgtctgg ag                                                22

<210>  5
<211>  23
<212>  DNA
<213>  Artificial

<220>
<223>  Synthetic DNA

<400>  5
gttttcaagg aattagacac tgc                                               23

<210>  6
<211>  23
<212>  DNA
<213>  Artificial

<220>
<223>  Synthetic DNA

<400>  6
caacagtctt ttgagtagca gtc                                               23

<210>  7
<211>  40
<212>  DNA
<213>  Artificial

<220>
<223>  Synthetic DNA

<400>  7
gatctccatg gctgatgaac tggcgttctc tcctcctttc                             40

<210>  8
<211>  40
<212>  DNA
<213>  Artificial

<220>
<223>  Synthetic DNA

<400>  8
tggcgctcga gccttgcacc ttcgggagcg ccgcgtgaag                             40

## Description

### Technical Field

The present invention relates to yeast for transformation, which is used for introduction of a target gene, a transformation method using the yeast for transformation, and a method for producing a substance using the yeast for transformation.

### Background Art

Yeast is broadly used not only as a model organism in the fields of genetic engineering, molecular biology, and the like, but also in fermented food industries or substance production industries using fermentation. Production of a useful substance using yeast begins from introduction of various genes involved in production of the useful substance into yeast via gene recombination techniques, so as to produce transformed yeast. High-level expression of a gene to be introduced is important for the productivity of the useful substance. Accordingly, if multiple such genes can be introduced, it can be said that the resultant would be useful as transformed yeast having good productivity for the useful substance.

Meanwhile, in gene recombination techniques, a selection marker is essential for introduction of a target gene. As selection markers, phenotypes such as drug resistance and auxotrophy are used. When drug resistance is used as a selection marker, a drug resistance gene is introduced together with a target gene into a host, so that the transformant becomes capable of growing even in a drug-containing medium. However, drug resistance genes do not always allow clear determination, since host cell sensitivity to drugs varies. Thus, it cannot be said that such drug resistance genes are more practical than auxotrophic markers. Also, drug resistance genes are heterologous genes. Hence, safety evaluation should be performed based on the law of Cartagena, followed by evaluation and prescribed procedures. When auxotrophy is used as a selection marker, first, a host is prepared by imparting auxotrophy thereto, so that the host is capable of growing only in a medium containing a predetermined nutrient, for example. Specifically, auxotrophy is imparted by disrupting the original gene of the host involved in synthesis of a nutrient. Then the gene involved in synthesis of the nutritional component is introduced together with a target gene into the host, so that it becomes possible for the host to grow even in a medium lacking the nutrient. As described above, a transformant into which a target gene has been introduced can be selected using changes such as imparting of drug resistance or disappearance of auxotrophy as an index.

JP Patent Publication (Kokoku) No. 7-114689 B (1995) discloses the construction of uracil auxotrophic yeast, tryptophan auxotrophic yeast, uracil and tryptophan auxotrophic yeast through disruption of a URA3 gene pair and/or a TRP1 gene pair in practical yeast (diploid) having a pair-of-chromosome structure. However, if they are used as hosts for transformation as disclosed in this patent document, only one copy of a target gene can be introduced, and multiple such genes cannot be introduced. Moreover, if yeast to which 2 types of auxotrophy have been imparted is used, only a maximum of 2 copies of a target gene can be introduced. Also, such a technique for disrupting a gene pair as disclosed in this patent document involves many experimental steps and many operational difficulties. Hence, the technique cannot be applied to all types of yeast and is not practical.

On the other hand, Applied and Environmental Microbiology, May 2005, p. 2789-2792 discloses a technology that involves construction of a strain (OC-2T strain) through imparting of tryptophan auxotrophy to wine yeast having homothallic properties (*Saccharomyces cerevisiae* OC-2 strain) and then introducing a target gene using the strain into a pair of chromosomes. Specifically, after introduction of such a target gene using tryptophan auxotrophy as an index, the target gene is introduced into each chromosome using the homothallic properties of the yeast. However, the OC-2T strain disclosed in this non-patent document contains only one type of selection marker, so that in the case of the OC-2T strain, only a maximum of 2 copies of the target gene can be introduced because of the auxotrophic marker.

### Disclosure of the Invention

### Problems to Be Solved by the Invention

As described above, only a maximum of 2 copies can be introduced in the case of an auxotrophic marker, when a target gene is introduced into yeast as a host. Alternatively, when a drug resistance marker is used, the number of copies can be increased, but it cannot be said that the use of a drug resistance marker is desirable, in view of unclear determination of marker gene introduction, working efficiency within laboratories (e.g., safety evaluation of foreign genes), and practical application of developed yeast. Under such circumstances, an object of the present invention is to provide yeast for transformation, which enables introduction of greater number of copies of a target gene or introduction of greater number of types of target genes, a transformation method, a method for producing a substance using the yeast for transformation, and the like.

### Means for Solving the Problems

As a result of intensive studies to achieve the above object, the present inventors have discovered that convenient introduction of a multiple-copy gene into genome is possible with the use of yeast having homothallic properties and succeeded in development of a new technology by which a plurality of selection markers can be provided to even high-order polyploid yeast. Thus, the present inventors have completed the present invention. Therefore, the present inventors have succeeded in provision of a plural number of types of selection markers, which has been difficult to achieve by the technique disclosed in the above-mentioned patent document, by providing such selection markers to yeast having homothallic properties using a so-called marker recycling method.

The present invention encompasses the following (1) to (16).
(1) Yeast for transformation, which is prepared by introducing a plurality of selection markers into yeast having homothallic properties.
(2) The yeast for transformation according to (1), wherein the yeast has spore-forming ability.
(3) The yeast for transformation according to (2), wherein the spore-forming ability is 0.1 % or higher.
(4) The yeast for transformation according to (1), wherein the selection marker is auxotrophy.
(5) The yeast for transformation according to (1), wherein the selection markers are two or more types of auxotrophy selected from the group consisting of uracil auxotrophy, histidine auxotrophy, and tryptophan auxotrophy.
(6) The yeast for transformation according to (1), wherein the yeast is a *Saccharomyces cerevisiae* OC-2 strain (NBRC2260).
(7) A transformation method, comprising the steps of:
   introducing a target gene to the yeast for transformation according to any one of (1) to (6) above; and
   selecting a strain, in which the target gene has been introduced, based on the selection markers in the yeast for transformation, whereby
   multiple target genes are introduced using the homothallic properties of the yeast for transformation.
(8) The transformation method according to (7), wherein the step of introducing the target gene and the step of selecting a strain in which the target gene has been introduced are performed more than once according to the number of introduced selection markers.
(9) The transformation method according to (7), further comprising the steps of:
   inducing spore formation in a spore formation medium after selecting a strain in which the target gene has been introduced; and culturing asci isolated from the spore formation medium in a germination medium.
(10) A method for producing a substance, comprising the step of culturing transformed yeast prepared by introducing multiple target genes into the yeast for transformation according to any one of (1) to (6) above.
(11) The method for producing a substance according to (10), wherein the target gene is a β-glucosidase gene and an object to be produced is ethanol that is produced using an oligosaccharide as a substrate.
(12) The method for producing a substance according to (10), wherein the target gene is a lactate dehydrogenase gene and an object to be produced is lactic acid.
(13) The method for producing a substance according to (10), wherein the target gene is expressed under the control of a pyruvate decarboxylase gene (PDC1) promoter.
(14) A method for providing multiple selection markers to yeast having homothallic properties, comprising the steps of:
   introducing a DNA fragment to yeast having homothallic properties and being imparted auxotrophy by deficiency of an auxotrophy-related gene involved in synthesis of a predetermined nutritional component, wherein the DNA fragment comprises an upstream region of a deletion-target auxotrophy-related gene that is involved in synthesis of a nutritional component differing from the predetermined nutritional component, the auxotrophy-related gene, and a downstream region of the deletion-target auxotrophy-related gene in such order;
   identifying a strain into which the DNA fragment has been introduced using the auxotrophy as an index;
   selecting a strain to which auxotrophy has been imparted again after the loss of the above auxotrophy-related gene, from among strains identified in the above steps;
   inducing spore formation by inoculating the strain selected in the above step in a spore formation medium; and
   culturing asci isolated from the spore formation medium in a germination medium.
(15) The method for providing multiple selection markers to yeast having homothallic properties according to (14), wherein the nutritional component is an amino acid or a base and the auxotrophy-related gene is an amino acid synthesis-related gene or a base synthesis-related gene.
(16) The method for providing multiple selection markers to yeast having homothallic properties according to (14), wherein the nutritional component is tryptophan, histidine, or uracil.

### Effects of the Invention

According to the yeast for transformation and the transformation method according to the present invention, a greater number of copies of a target gene can be introduced compared with conventional cases, or a greater number of types of target genes can be introduced compared with conventional cases. Therefore, transformed yeast expressing a target gene at a high level and transformed yeast expressing a greater number of types of a target gene(s) can be developed.

Also, according to the method for producing a substance according to the present invention, compared with cases in which conventional yeast for transformation is used, productivity of a target substance can be drastically improved and the cost required for substance production can be significantly reduced.

Furthermore, according to the method for preparing yeast for transformation according to the present invention, yeast for transformation that enables introduction of a greater number of copies of a target gene compared with conventional cases, or introduction of a greater number of types of target genes compared with conventional cases can be prepared very conveniently.

This description includes part or all of the contents as disclosed in the description and/or drawings of Japanese Patent Application No. 2007-200978, from which the present application claims a priority.

### Brief Description of the Drawings

Fig. 1 schematically shows the steps for construction of yeast for transformation having homothallic properties and a plurality of selection markers through application of the present invention.
Fig. 2 shows characteristic graphs showing the results of measuring the relationship between sugar concentrations in medium and the expression levels of a TDH1 gene, a TDH3 gene, and a PDC1 gene in the YPH500 strain and the OC-2T strain.
Fig. 3 explains the process of construction of pIBG13 from pIHCS and pBG211.
Fig. 4 explains the process of construction of pIWBGL1 from pRS404 and pBlue-BGL1.
Fig. 5 explains the process of construction of pRS406BGL1 from pRS406 and pIWBGL1.
Fig. 6 is a characteristic figure showing the results of comparing PNPG activity among the OC-2HU strain, the OC-2ABGL2 strain, and the OC-2ABGL4 strain.
Fig. 7 is a characteristic figure showing the results of comparing ethanol formation potential among the OC-2HU strain, the OC-2ABGL2 strain, and the OC-2ABGL4 strain.

### Best Mode for Carrying Out the Invention

The present invention will be further explained with reference to drawings and examples.

The yeast for transformation according to the present invention is prepared by introducing a plurality of selection markers into yeast having homothallic properties. Here, the term "yeast for transformation" refers to objective yeast into which a target gene is introduced, which differs from transformed yeast into which such a target gene has been introduced.

Also, the term "yeast having homothallic properties" is synonymous with the term "homothallic yeast." In such yeast having homothallic properties, sexual conversion takes place due to endonuclease encoded by an HO gene and then daughter cells with different sex germinate from monoploid mother cells. Two types of sex (mating types), "a" cells and "α" cells, are present in monoploid yeast. No mating takes place between "a" cells or between "α" cells. The "a" cells and "α" cells secrete "a" factor and "α" factor, respectively, which are their unique sex hormones (pheromones). When they come sufficiently close to each other and sense each others' pheromones with the receptors on their cell membranes, they stop their general proliferation and begin mating. Cells elongate facing each other, their cell membranes and then nuclei become fused to each other, and then diploid cells are formed. Therefore, yeast having homothallic properties forms a life circle such that daughter cells that have germinated from monoploid mother cells undergo mating with the mother cells to form diploids. According to the life circle, monoploid yeast having homothallic properties is transformed into diploid yeast having completely the same genotype other than MAT genes (MATa gene and MATα gene) that determine the mating type. In addition, diploid yeast initiates meiosis under nutrient starvation conditions such as depletion of a nitrogen source in medium, so as to form spores. An yeast spore has two "a" cells and two "α" cells in its sac-like structure referred to as an "ascus." A spore germinates when nutrient starvation conditions are canceled and initiates proliferation again after germination as a monoploid.

Any yeast can be used as yeast having homothallic properties without particular limitations. An example of yeast having homothallic properties is, but is not limited to, the *Saccharomyces cerevisiae* OC-2 strain (NBRC2260). Further examples of yeast having homothallic properties include alcohol yeast (Tai-ken No. 396, NBRC0216) (Source: "Various properties of alcohol yeast" Shu-ken kaiho, No. 37, p. 18-22 (1998. 8)), and ethanol-producing yeast isolated in Brazil and Okinawa (Source: "Genetic properties of Saccharomyces cerevisiae wild-type strains isolated in Brazil and Okinawa" KAGAKU TO SEIBUTSU (Journal of the Japan Society for Bioscience, Bioteclmology, and Agrochemistry), Vol. 65, No. 4, p. 759-762 (1991. 4)), and 180 (Source: "Screening for yeast having strong alcohol fermentation ability" Journal of the Brewing Society of Japan, Vol. 82, No. 6, p. 439-443 (1987. 6)). Also, even yeast expressing a heterothallic phenotype can be used as yeast having homothallic properties by introducing an HO gene into the heterothallic yeast so that the gene can be expressed. Hence, the term "yeast having homothallic properties" in the present invention refers also to yeast in which the HO gene has been introduced so that it can be expressed.

Of these examples, the *Saccharomyces cerevisiae* OC-2 strain is preferable since it has been conventionally used for wine brewing and thus the safety thereof has been confirmed. Also, the *Saccharomyces cerevisiae* OC-2 strain is preferable, since the strain has good promoter activity under high sugar concentration conditions, as described later in Examples. Particularly, the *Saccharomyces cerevisiae* OC-2 strain is preferable, since the promoter activity of a pyruvate decarboxylase gene (PDC1) is excellent under high sugar concentration conditions.

In the yeast for transformation according to the present invention, a plurality of selection markers are provided. Specifically, a phenotype, in which a growth state under predetermined conditions to be observed distinctively from a growth state under other conditions due to loss of function of a given gene in host, is used as a selection marker. Here, the term, "loss of function of a given gene" refers to deletion of the gene, transcriptional suppression of the gene, translational suppression of the gene, mutation of the gene itself, or the like. In addition, in high-order polyploids, the genes located in all chromosomes lose their functions. More specifically, an example of such selection marker is auxotrophy or the like that is imparted as a result of loss of the functions of a gene involved in biosynthesis of a nutritional component essential for growth. Examples of auxotrophy include uracil auxotrophy that is imparted by loss of the functions of an URA3 gene in yeast, histidine auxotrophy that is imparted by loss of the functions of an HIS3 gene in yeast, and tryptophan auxotrophy that is imparted by loss of the functions of a TRP1 gene. In addition, examples of a gene, the functions of which are lost, include not only the URA3 gene, the HIS3 gene, and the TRP1 gene, but also a LUE2 gene, an ADE2 gene, and an LYS2 gene. Leucine auxotrophy can be imparted by loss of the functions of the LUE2 gene, adenine auxotrophy can be imparted by loss of the functions of the ADE2 gene, and lysine auxotrophy can be imparted by loss of the functions of the LYS2 gene.

To provide a plurality of selection markers, first, yeast having a single selection marker is prepared. In addition, this yeast has homothallic properties as described above. As such homothallic yeast having a single selection marker, the *Saccharomyces cerevisiae* OC-2T strain disclosed in Applied and Environmental Microbiology, May 2005, p. 2789-2792 can be used, for example. The *Saccharomyces cerevisiae* OC-2T strain is a homothallic yeast strain, which is prepared by deletion of a TRP1 gene pair in the *Saccharomyces cerevisiae* OC-2 strain known as wine yeast and has tryptophan auxotrophy imparted as a selection marker. Also, the *Saccharomyces cerevisiae* OC-2U strain disclosed in the same report can also be used. The *Saccharomyces cerevisiae* OC-2U strain is a homothallic yeast strain, which is prepared by deletion of a URA3 gene pair in the *Saccharomyces cerevisiae* OC-2 strain and has uracil auxotrophy imparted as a selection marker.

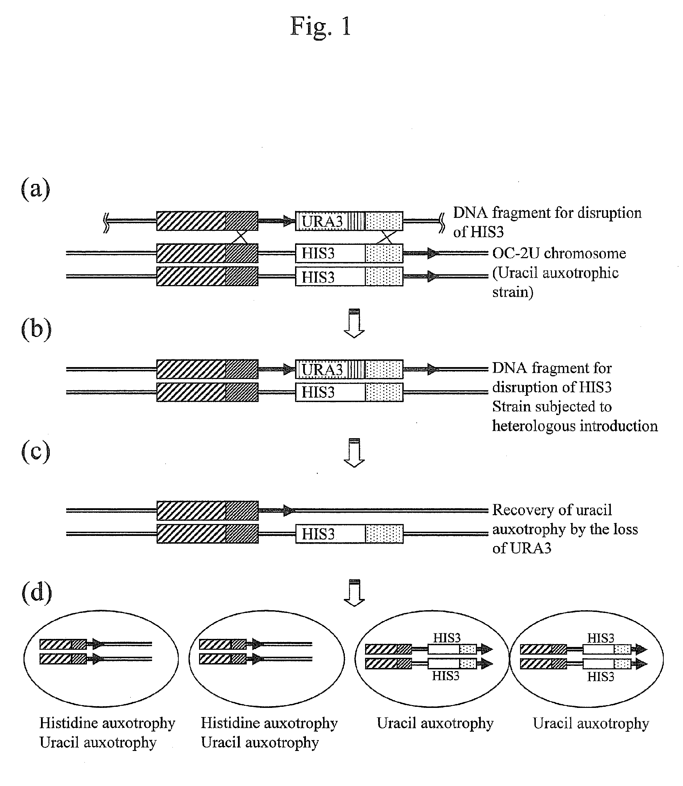

Next, the second selection marker is introduced as described below. When auxotrophy for a different nutritional component is imparted as such 2^{nd} selection marker, for example, genes involved in biosynthesis of the relevant nutritional component are deleted from all chromosomes. As an example, Fig. 1 schematically shows a method for imparting histidine auxotrophy as the 2^{nd} selection marker to homothallic yeast (OC-2U strain) having uracil auxotrophy imparted thereto. First, a DNA fragment for deletion of a HIS3 gene in a host is prepared. The DNA fragment has a structure wherein the upstream region of the HIS3 gene in the host, the URA3 gene, and the downstream region of the HIS3 gene in the host are linked in this order. Next, when the DNA fragment is introduced into homothallic yeast having uracil auxotrophy imparted thereto, homologous recombination with the host's chromosome takes place at the upstream and the downstream regions contained in the introduced DNA fragment, as shown in Fig. 1(a). Introduction of the DNA fragment into the chromosome causes disappearance of uracil auxotrophy due to expression of the URA3 gene. Therefore, yeast transformed with such DNA fragment can grow in medium containing no uracil, so that identification is possible using disappearance of uracil auxotrophy as an index. At this time, the chromosomal structure of such yeast transformed with the DNA fragment is as shown in Fig. 1(b), wherein the URA3 gene is inserted so that it is replaced by the HIS3 gene in one chromosome. The HIS3 gene in the other chromosome remains. Yeast at this stage does not exert auxotrophy, but exerts prototrophy.

Next, a strain that has lost the introduced URA3 gene is selected. Positive selection thereof can be carried out by culturing yeast from which uracil auxotrophy has disappeared in a medium containing 5-fluoroorotic acid (referred to as 5-FOA). Specifically, when yeast having an uracil biosynthesis system is cultured in the presence of 5-FOA, an uracil analog is synthesized using 5-FOA as a substrate and the yeast having the uracil biosynthesis system dies. Hence the strain that grows after culturing of yeast from which uracil auxotrophy has disappeared in a medium containing 5-FOA can be identified as a strain that has lost the URA3 gene from a chromosome. The chromosomal structure of such yeast that has lost the URA3 gene from the chromosome is as shown in Fig. 1(c), wherein only the HIS3 gene in one chromosome has been deleted.

Next, yeast having chromosomal configuration as shown in 1(c) is cultured in a spore formation medium, so as to cause spore formation via meiosis. A spore formation medium is not particularly limited and a medium having conventionally known composition can be used as the spore formation medium. Subsequently, spores (asci) are isolated from the medium using an instrument such as a micromanipulator. An isolated ascus contains two spores (monoploids) containing at least one chromosome of a chromosome pair shown in Fig. 1(c) and two spores (monoploids) containing the other chromosome. Next the spores are cultured in a germination medium, so that each spore undergoes vegetative-growth life circle. At this time, yeast having homothallic properties undergoes a life circle such that daughter cells that have germinated from monoploid mother cells undergo mating with the mother cells to form diploids. Hence, the cells are transformed into diploid yeast having completely the same genotype other than MAT genes (MATa gene and MATα gene) that determine the mating type. Therefore, 2 types of yeast having the chromosomal configuration shown in Fig. 1(d) can be obtained by causing germination of homothallic yeast having the chromosomal configuration shown in Fig. 1(c) after spore formation. One yeast diploid exerts uracil auxotrophy alone, but the other yeast diploid exerts histidine auxotrophy imparted, in addition to uracil auxotrophy. Hence, homothallic yeast to which histidine auxotrophy has been imparted as the 2^{nd} selection marker can be prepared as a strain that grows in a medium containing uracil and histidine, but is unable to grow in a medium lacking uracil and histidine.

In addition, it is needless to explain that the 3^{rd} selection marker can be provided through repetition of the above-mentioned technique. Specifically, a plurality of selection markers can be successively provided to yeast having homothallic properties using the above-mentioned technique. The above-mentioned technique is a method that effectively uses a characteristic life circle such as homothallic properties and a phenomenon such as the loss of the URA3 gene. In addition, a system is explained in the above explanation, wherein the URA3 gene is used for a DNA fragment for deletion of the HIS3 gene and 5-FOA is used for determining the loss of the URA3 gene. A deletion-target gene (to be deleted) is not limited to the HIS3 gene, but a marker for marker recycling is preferably the URA3 gene. This is because 5-FOA that enables selective obtainment of an uracil auxotrophic strain is used. In addition, the above DNA fragment for deletion of the HIS3 gene is also disclosed in the paper of Rinji Akada et al., for the purpose of using it for a marker recycling method for haploid strains (Yeast, Volume 23, Issue 5, Pages 399-405).

The thus constructed yeast having homothallic properties, to which a plurality of selection markers have been provided, can be used for transformation using the selection markers. Hence, the yeast can be broadly used as yeast for transformation. The yeast for transformation according to the present invention is handled as "self-cloning" in the law of Cartagena, so that the use thereof is convenient even in view of safety evaluation for genetically engineered organisms. Various target genes can be introduced into the yeast for transformation without limitations. Here, an example of a target gene is a gene for imparting ability of producing a useful substance. Examples of such target gene include a lactate dehydrogenase gene (see Applied and Environmental microbiology, May 2005, p. 2789-2792) for imparting ability for generating lactic acid to yeast, a glucoamylase gene for imparting ability of directly generating glucose from starch to yeast (see Journal of Fermentation and Bioengineering, Vol. 81, Issue 2, 1996, pages 98-103), and a BGL gene that is one of cellulase genes disclosed in the present invention.

As a method for introducing a target gene, any conventional technique known as a yeast transformation method can be applied herein. Specifically, introduction can be carried out by electroporation "Meth. Enzym., 194, p. 182 (1990)," a spheroplast method "Proc. Natl. Acad. Sci. U.S.A., 75 p. 1929 (1978)," and a lithium acetate method, "J. Bacteriology, 153, p. 163 (1983)," Proc. Natl. Acad. Sci. U.S.A., 75 p. 1929 (1978), Methods in yeast genetics, 2000 Edition: A Cold Spring Harbor Laboratory Course Manual, or the like, but the examples are not limited thereto.

Also, promoters or terminators for regulation of gene expression of a target gene can be used without limitations. Gene introduction can be carried out using a plasmid containing a target gene, a promoter, and a terminator. As a promoter, a glyceraldehyde-3-phosphate dehydrogenase (TDH3) gene promoter, a 3-phosphoglycerate kinase gene (PGK1) promoter, or the like can be used. Particularly a pyruvate decarboxylase gene (PDC1) promoter is preferable because of its high ability of causing high-level expression of the downstream target gene.

When a target gene is introduced into yeast having homothallic properties, one of a plurality of selection markers that have been provided to the yeast is used. When a selection marker to be used herein is auxotrophy, a gene that causes disappearance of the auxotrophy is introduced together with the target gene. A strain can be selected using disappearance of the auxotrophy as an index. Also, thereafter, the asci of the strain are isolated and then monoploid spores are cultured in germination medium. Therefore, the yeast having homothallic properties exerts a life circle such that daughter cells that have germinated from monoploid mother cells undergo mating with the mother cells, forming diploids. Specifically, the yeast is transformed into diploid yeast having completely the same genotype other than MAT genes that determine the mating type (MATa gene and MATα gene). Hence, the target gene is introduced into all chromosomes in a manner such that multiple copies of the target gene are introduced through single transformation into chromosomes. In contrast, when heterothallic yeast is used, only one copy of the target gene can be introduced through single transformation.

Furthermore, a plurality of selection markers are provided by a convenient technique to the above-prepared yeast for transformation, so that a plurality of target genes can be introduced according to the number of selection markers. At this time, a plurality of target genes may all be the same genes or may be different genes. When the same genes are introduced, transformed yeast capable of expressing the genes at unprecedentedly high levels can be obtained. Specifically, when homothallic yeast for transformation having 2 types of selection markers is used, a maximum of 4 copies of a target gene can be introduced. Similarly, when homothallic yeast for transformation having 3 types of selection markers is used, a maximum of 6 copies of a target gene can be introduced.

### Examples

Hereafter, the present invention is described in greater detail with reference to the examples, although the technical scope of the present invention is not limited thereto.

### [Example 1]

In this Example, the *Saccharomyces cerevisiae* OC-2U strain (Saito et al., Journal of Ferment. Bioeng. 81: 98-103 (1996)) prepared by imparting uracil auxotrophy to a *Saccharomyces cerevisiae* OC-2 strain was used as a host. Histidine auxotrophy was further imparted as a 2^{nd} selection marker (see Figs. 1 (a) to (d)).

First, a DNA fragment for disruption of HIS3 described in Akada et al., Yeast 2006 23: 399-405 (PCR fragment that consisted of a URA3 marker attached to a 40-base-repeated-generating sequence flanked by the HIS3 targeting sequence at both ends) was prepared. The OC-2U strain was transformed using a Frozen EZ transformation kit II: Zymo Research and then colonies that had grown on an SD plate were isolated. On such SD medium plate, uracil auxotrophic strains cannot grow, but strains wherein uracil auxotrophy has disappeared can grow. Hence, transformants were selected using as an index the disappearance of uracil auxotrophy resulting from the expression of the URA3 gene contained in the DNA fragment for disruption of HIS3.

Next, the thus obtained colonies were separated into single colony on SD plates and then cultured in YPD liquid media at 30°C for 1 day. The culture solution (0.2 ml) was spread on an FOA plate. Here, the FOA plate is composed of Bacto yeast nitrogen base (0.67%), glucose (2%), uracil (50 µg/ml), and 5-FOA (0.1 %). Colonies grown on the FOA plate were strains that had lost the URA3 gene contained in the introduced gene fragment and recovered the OC-2U strain's original uracil auxotrophy.

Next, colonies that had grown on the FOA plate were pre-cultured on an YPD plate at 30°C for 1 day. Subsequently, they were inoculated on a Sherman plate, which is a spore formation medium, followed by 3 days of culture at 25°C. Spore formation was then confirmed. Spore isolation was carried out using a micromanipulator (NARISHIGE).

The thus obtained asci contained 3 or 4 spores. The OC-2U strain originally has homothallic properties, such that it has an HO gene undergoing sexual conversion of the yeast. After meiosis, monoploid spores become diploid cells as they become vegetative cells. Accordingly, 2 out of 4 spores of the transformed strain lost the URA3 gene, the HIS3 gene was disrupted, and uracil auxotrophy was recovered. Thus, the strain was isolated as having both uracil auxotrophy and histidine auxotrophy. The 2 remaining spores were of the strain having uracil auxotrophy alone, since they had normal HIS3 genes.

Therefore, the 4 thus obtained spores were separately cultured on 4 types of plates: SD plate, SD plate + uracil (20 mg/L), SD plate + histidine (20 mg/L), and SD plate + uracil + histidine. As a result, a strain capable of growing only on the SD + uracil + histidine plate could be successfully isolated. Therefore, the *Saccharomyces cerevisiae* OC-2U strain to which histidine auxotrophy had been imparted in addition to uracil auxotrophy could be successfully prepared. The homothallic yeast prepared in this Example, having both uracil auxotrophy and histidine auxotrophy, was named the *Saccharomyces cerevisiae* OC-2HU strain.

### [Example 2]

In this Example, it was verified that strains derived from the *Saccharomyces cerevisiae* OC-2 strain were excellent in substance productivity. Specifically, when a heterologous gene is introduced via transformation to achieve high substance productivity, there is a need to actively use high expression promoters. Promoters include constitutive promoters and inducible promoters. When the purpose is to achieve high productivity, there is a need to select a promoter with a high expression level at a high sugar concentration, regardless of promoter type. Therefore, the YPH500 strain (laboratory yeast) and the OC-2T strain (Saito et al., Journal of Ferment. Bioeng. 81: 98-103 (1996)) were examined as follows. Specifically, a TDH1 promoter, a TDH3 promoter, and a PDC1 promoter (which are generally used for yeast gene recombination techniques) were studied for promoter activity by comparing the expression levels of genes transcribed by the promoters.

First, the YPH500 strain and the OC-2T strain were shake-cultured in a YPD liquid culture medium using 2 levels of glucose in terms of amount (2% or 10%). The thus cultured cells were sampled and then cDNA was prepared using a 1^{st} strand cDNA synthesis kit for RT-PCR (Roche). The expression levels of the TDH1 gene, the TDH3 gene, and the PDC1 gene were determined by quantitative PCR using the following primers and Roche Light cycler 330. Table 1 shows the primer sequences used herein.

**Table 1**

| Gene detected | Primer name | Sequence | |
|---|---|---|---|
| PDC1 | PDC1+926H: | tccactccgaccacatgaag | SEQ ID NO: 1 |
| | PDC1+1092H | tggggtagaagctgggacag | SEQ ID NO: 2 |
| TDH1 | TDH1 F | ttctctaaagatcgatgtcgc | SEQ ID NO: 3 |
| | TDH1 R | gagacaatcttcttgtctggag | SEQ ID NO: 4 |
| TDH3 | TDH3 F | gttttcaaggaattagacactgc | SEQ ID NO: 5 |
| | TDH3 R | caacagtcttttgagtagcagtc | SEQ ID NO: 6 |

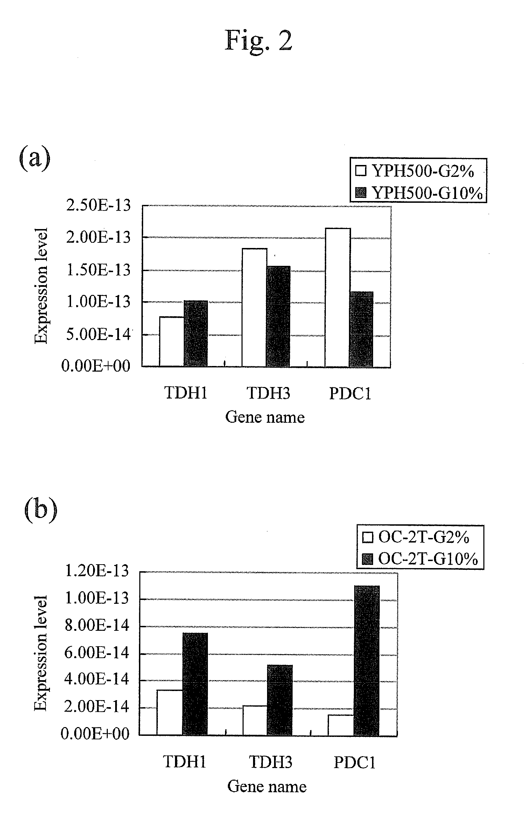

Also, Fig. 2 (a) and Fig. 2 (b) show the expression levels of the TDH1 gene, the TDH3 gene, and the PDC1 gene in the YPH500 strain and the OC-2T strain, respectively, resulting from different glucose concentrations. The YPH500 strain, which is a laboratory yeast strain, showed a decreasing tendency for all expression levels of the TDH3 gene and the PDC1 gene subjected to the experiment, when the glucose concentration had been increased from 2% to 10%. In contrast, the OC-2T strain showed an increasing tendency for all the expression levels of the TDH1 gene, the TDH3 gene, and the PDC1 gene, when the glucose concentration had been increased from 2% to 10%. Hence, it was considered based on the results that the YPH500 strain capable of exerting sufficient performance was selected in the case of general YPD medium containing 2% glucose since it has been originally used as laboratory yeast; in contrast, the results support the fact that the OC-2 strain could easily exert its performance in a medium with high sugar concentration, since it has conventionally been used for wine brewing and the like in Japan.

In view of the above results and considerations, it can be said that the OC-2HU strain prepared in Example 1 is useful as practical yeast having good substance productivity.

### [Example 3]

In this Example, spore formation rate of the OC-2HU strain prepared in Example 1 was confirmed. In addition, a spore formation rate of 0.1% or more is preferable, since this facilitates ascus isolation using a micromanipulator, as explained in the following Examples. An YPD plate containing 2% glucose was streaked with the OC-2HU strain prepared in Example 1 and then the strain was cultured at 30°C for 1 day. Cultured cells were transferred to a Sherman plate, which is a spore formation medium, and then cultured at 25°C for 3 to 4 days. Spore formation was confirmed under a microscope. Table 2 shows spore formation percentages of the OC-2HU strain. In addition, spore formation percentage was calculated according to the following formula. Spore formation percentage = (number of spores) × 100/(cell count)

**Table 2**

| Days for culture | (1) Spore formation rate | (2) Cell count | (3) Number of spores |
|---|---|---|---|
| Day 1 | 0 % | 150 | 0 |
| Day 2 | 0.9 % | 540 | 5 |
| Day 3 | 10.1 % | 495 | 50 |

It was clarified based on the results of the Example that in the case of the OC-2HU strain prepared in Example 1, ascus isolation could be carried out using a micromanipulator from about day 2 after the initiation of culture.

### [Example 4]

In this Example, the OC-2HU strain prepared in Example 1 was used as yeast for transformation and 4 copies of a cell-surface-display BGL gene were introduced as target genes.

### (1) Preparation of a strain into which 2 copies of Arming BGL are introduced

First, the OC-2HU strain prepared in Example 1 was transformed with a plasmid prepared through linearization of pIBG13 (HIS3 marker), the Arming BGL-DNA constructed as described below, with restriction enzyme *Nde* I, using Frozen-EZ Yeast Transformation II (Zymo Research). SD + Histidine plates were used as selection plates. Colonies obtained by transformation were separated on the same plates to eliminate false-positive strains.

Next, the thus obtained colonies were cultured on a YPD plate at 30°C for 1 day. The spore formation medium (Sherman) was streaked with cultured cells and then the resultant was subjected to plate culture at 25°C. Spore formation was confirmed on day 3 of culture. Four spores contained in asci were isolated using a micromanipulator (NARISHIGE). A strain into which 2 copies of pIBG13 had been introduced and a strain that had lost pIBG13 were separated at a ratio of 2 : 2. Hence, the thus obtained spores were cultured in SD medium at 30°C for 2 days, and then a strain from which histidine auxotrophy had disappeared was identified. The thus obtained strain was named OC-2ABGL2, into which 2 copies of pIBG13 had been introduced.

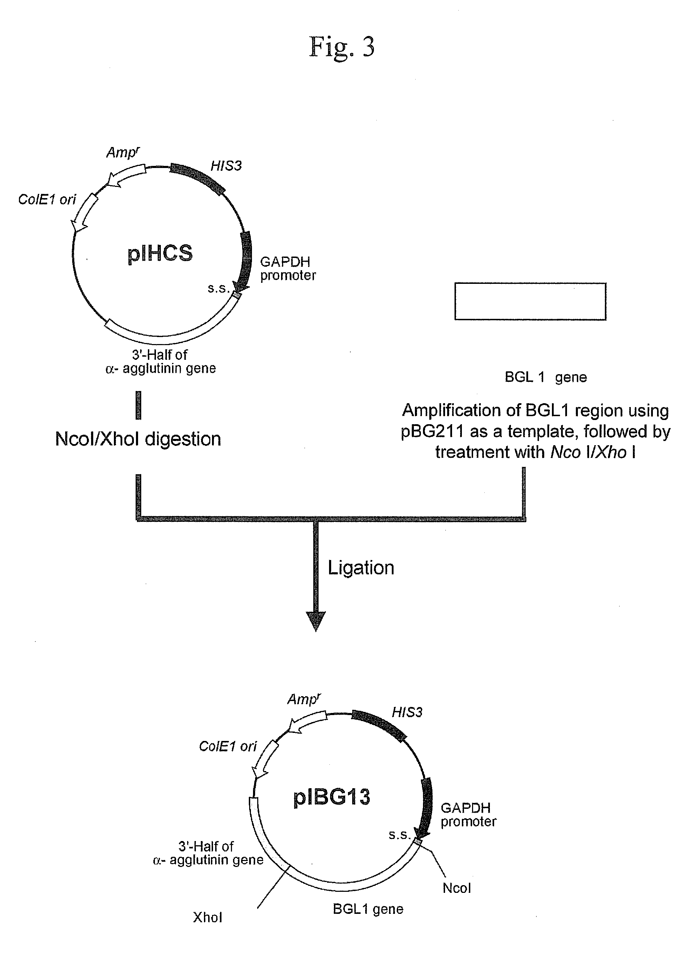

In addition, as shown in Fig. 3, pIBG13 was constructed from pIHCS (see Yasuya Fujita et al., "Direct and Efficient Production of Ethanol from Cellulosic Material with a Yeast Strain Displaying Cellulolytic Enzymes" Appl Environ Microbiol. 2002 October; 68 (10): 5136-5141) and pBG211 (see Murai, T et al., "Assimilation of cellooligosaccharides by a cell surface-engineered yeast expressing β-glucosidase and carboxymethylcellulase from Aspergillus aculeatus." Appl. Environ. Microbiol. 1998 64: 4857-4861). Specifically, a BGL1 fragment (2.5 kbp) having an *Nco* I/*Xho* I site on each end was amplified by PCR using pBG211 as a template and the following primer pair set.
5'-GATCTCCATGGCTGATGAACTGGCGTTCTCTCCTCCTTTC-3'(SEQ ID NO: 7)
5'-TGGCGCTCGAGCCTTGCACCTTCGGGAGCGCCGCGTGAAG-3'(SEQ ID NO: 8)

The thus amplified BGL1 fragment was treated with *Nco* I/*Xho* I. Also, pIHCS was treated with *Nco* I/*Xho* I and then the resultant was ligated to the BGL1 fragment subjected to treatment with *Nco* I/*Xho* I, thereby constructing pIBG13.

### (2) Preparation of a strain into which 4 copies of Arming BGL are introduced

OC-2ABGL2 prepared in (1) above was transformed with a plasmid prepared by linearization of pRS406BGL1 (URA3 marker) (constructed as described below) with restriction enzyme *Nde* I, using Frozen-EZ Yeast Transformation II (Zymo Research). SD plates were used as selection plates. Colonies obtained by transformation were separated on the same plates to eliminate false-positive strains.

Next, the thus obtained colonies were cultured on a YPD plate at 30°C for 1 day. A Sherman plate, which is a spore formation medium, was streaked with cultured cells and then the resultant was subjected to plate culture at 25°C. Spore formation was confirmed on day 3 of culture and then 4 spores contained in asci were isolated using a micromanipulator (NARISHIGE). Subsequently, the thus obtained spores were cultured in SD medium at 30°C for 2 days, so that a strain from which uracil auxotrophy had disappeared was identified. The thus identified strain was named OC-2ABGL4, wherein a total of 4 copies of Arming BGL gene fragment (2 copies of pIBG13 and 2 copies of pRS406BGL1) had been introduced into the genome.

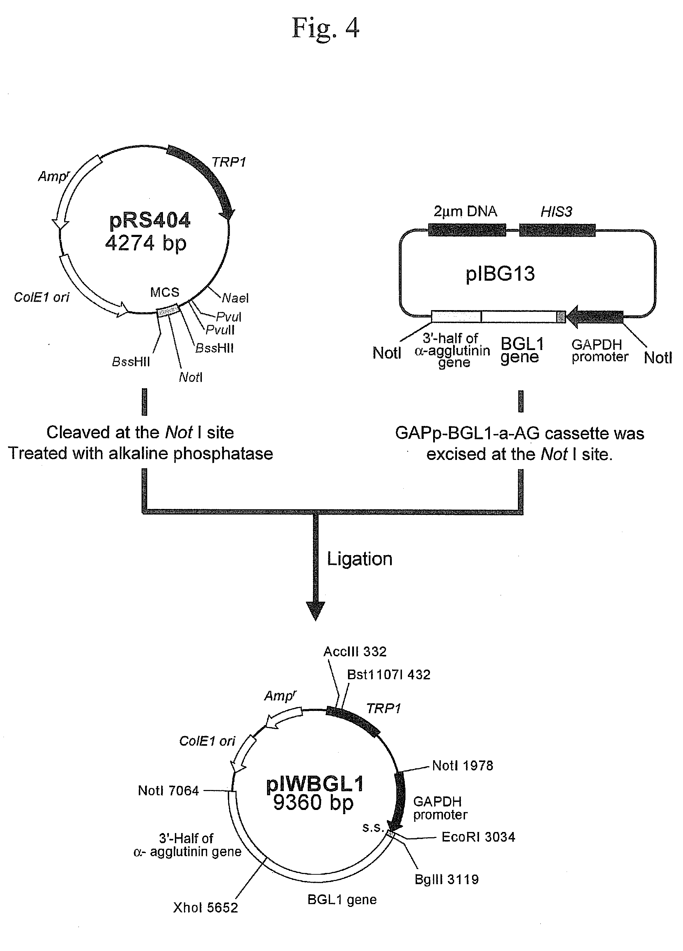

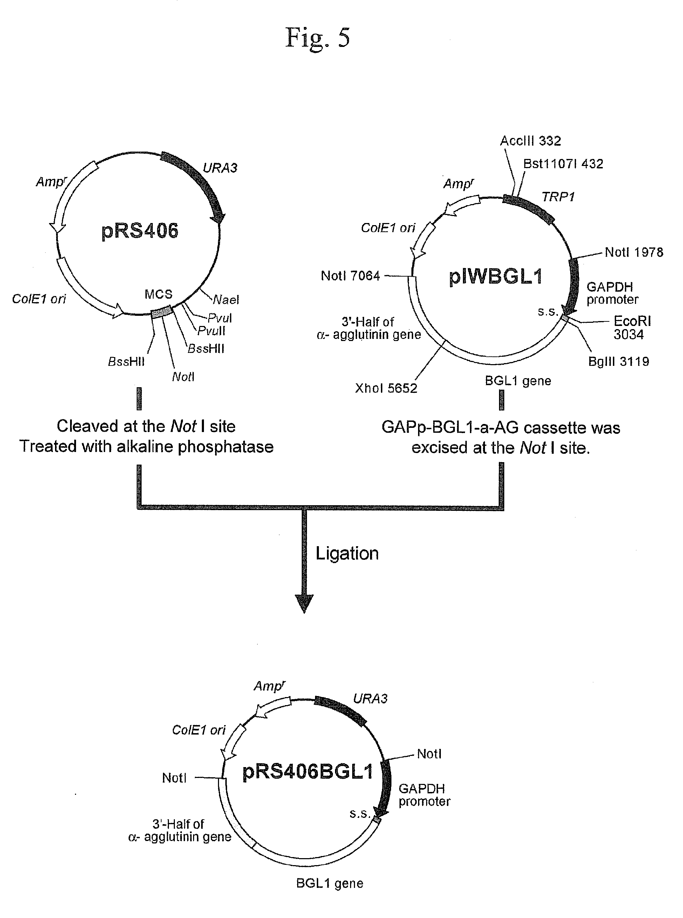

In addition, pRS406BGL1 was, as shown in Figs. 4 and 5, constructed from pIWBGL1 constructed from pRS404 (ATCC Number: 87515) and pIBG13 and pRS406 (ATCC Number: 87517). When pIWBGL1 was constructed, first, pRS404 was treated with *Not* I and then treated with alkaline phosphatase. Then pIBG13 was treated with *Not* I, so that the GAPDH promoter-BGL1 gene-3'half of an a-agglutinin gene was excised. Then the resultant was ligated to pRS404 that had been treated with *Not* I and then with alkaline phosphatase. Therefore, pIWBGL1 was constructed.

Next, when pRS406BGL1 used in this Example was constructed, pIWBGL1 was treated with *Not* I, so that the GAPDH promoter-BGL1 gene-3'half of an a-agglutinin gene was excised. The resultant was ligated to pRS406 that had been treated with *Not* I and then with alkaline phosphatase. Therefore, pRS406BGL1 was constructed.

### (3) Determination of p-nitrophenyl-β-D-glucoside (PNPG) activity

Next, OC-2ABGL2 constructed in (1) above and OC-2ABGL4 constructed in (2) above were evaluated for the activity of β-glucosidase that is the BGL gene product. Specifically, since β-glucosidase causes the generation of p-nitrophenol and D-glucose using PNPG as a substrate, the amount of p-nitrophenol was measured (PNPG activity) for evaluation. The amount of an enzyme that causes the generation of one 1 micromole of p-nitrophenol per minute under the following conditions was defined as 1 U.

Reagents used for measurement are as follows.
- 0.1 M acetate buffer pH 5.0 (25°C)
- 20 mM aqueous PNPG solution (603 mg of PNPG was dissolved in 100 ml of distilled water)
- 0.2 M Na₂CO₃ solution (21.2 g of anhydrous sodium carbonate was dissolved in distilled water)

Procedures for measurement are as follows. First, a mixed solution for reaction (1.0 ml of 0.1 M acetate buffer and 0.5 ml of aqueous PNPG solution) was prepared in a test tube and then pre-heated at 37°C for approximately 5 minutes. Next, 0.5 ml of a diluted culture solution was added and then reaction was initiated. Next, reaction was accurately carried out at 37°C for 15 minutes, and 2 ml of an Na₂CO₃ solution was added to stop the reaction. Subsequently, absorbance in the reaction solution was measured at 400 nm. A solution for a blind test was prepared by allowing the reaction solution to stand at 37°C for 15 minutes, adding 2 ml of an Na₂CO₃ solution, mixing the solution, and then adding 0.5 ml of an enzyme solution.

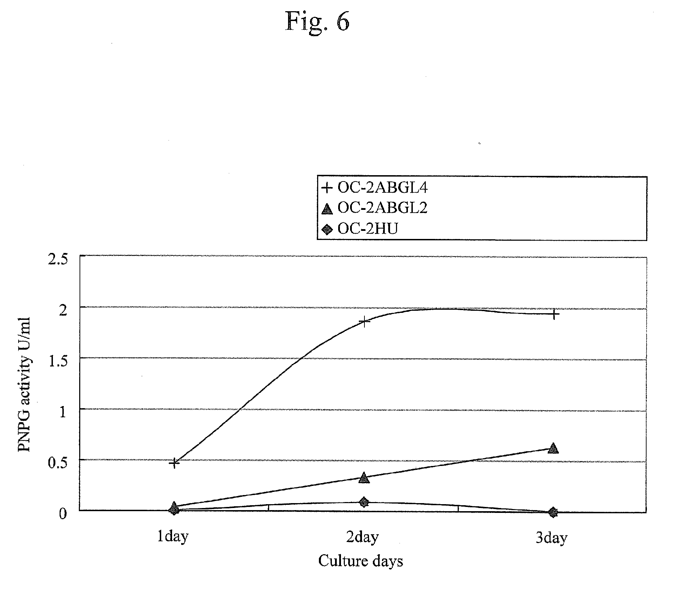

In addition, the following solution was prepared in this Example as a diluted culture solution. Specifically, first, the culture composition in a YPD medium was adjusted, so as to achieve OD660 = 20. OC-2ABGL2 constructed in (1) above or OC-2ABGL4 constructed in (2) above was inoculated on medium supplemented with cellobiose 5%, yeast extract 1%, and peptone 2% to a total volume of 20 ml. Shake culture was carried out under conditions of 30°C and 60 rpm. On days 1, 2, and 3 after the start of culture, 1 ml of the medium was collected, diluted 50 times, and then subjected to the experiment. Fig. 6 shows the results.

As is clear from Fig. 6, whereas PNPG activity was not detected in the parent strain (OC-2HU strain) into which no Arming BGL gene had been introduced, PNPG activity was significantly improved as the number of Arming BGL genes increased.

### [Example 5]

In this Example, OC-2ABGL2 and OC-2ABGL4 prepared in Example 3 were evaluated for ethanol formation potential. First, the culture composition in a YPD medium was adjusted, so as to achieve OD660 = 20. A medium supplemented with cellobiose (5%), yeast extract (1%), and peptone (2%) was seeded with OC-2ABGL2 prepared in Example 3 (1) or OC-2ABGL4 prepared in Example 3 (2). Shake culture was then performed under conditions of 30°C and 60 rpm.

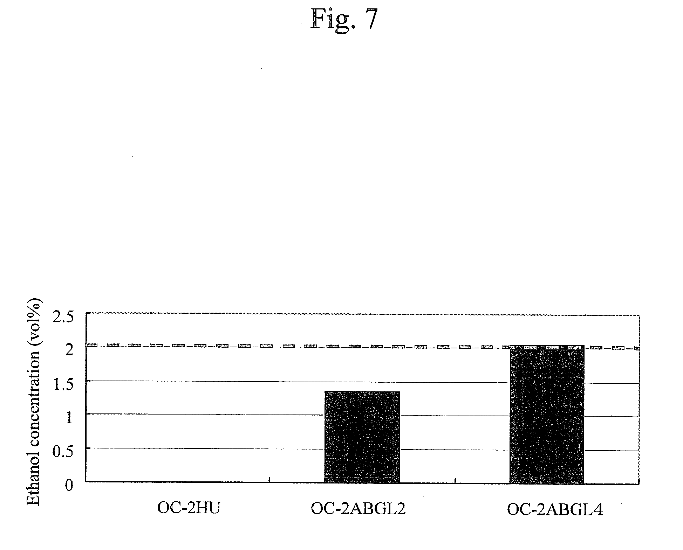

Subsequently, the concentration of ethanol contained in the culture solution was measured on day 1 after the start of culture. Ethanol concentration was measured using an enzyme sensor (Oji Scientific Instruments, Model No. BF4). Fig. 7 shows the results of the measurement.

As is clear from Fig. 7, OC-2ABGL4 could synthesize 2% by volume of ethanol from 4% cellobiose contained in a medium on day 1 of culture. BGL activity could be confirmed to an extent such that the ability of ethanol fermentation using cellobiose (which OC-2HU does not originally possess) as a substrate could be imparted.

All publications, patents, and patent applications cited in this specification are herein incorporated by reference in their entirety.

## Claims

1. Yeast for transformation, which is prepared by introducing a plurality of selection markers into yeast having homothallic properties.

2. The yeast for transformation according to claim 1, wherein the yeast has spore-forming ability.

3. The yeast for transformation according to claim 2, wherein the spore-forming ability is 0.1 % or higher.

4. The yeast for transformation according to claim 1, wherein the selection marker is auxotrophy.

5. The yeast for transformation according to claim 1, wherein the selection markers are two or more types of auxotrophy selected from the group consisting of uracil auxotrophy, histidine auxotrophy, and tryptophan auxotrophy.

6. The yeast for transformation according to claim 1, wherein the yeast is a *Saccharomyces cerevisiae* OC-2 strain (NBRC2260).

7. A transformation method, comprising the steps of:
introducing a target gene to the yeast for transformation according to any one of claims 1 to 6; and
selecting a strain, in which the target gene has been introduced, based on the selection markers in the yeast for transformation, whereby
multiple target genes are introduced using the homothallic properties of the yeast for transformation.

8. The transformation method according to claim 7, wherein the step of introducing the target gene and the step of selecting a strain in which the target gene has been introduced are performed more than once according to the number of introduced selection markers.

9. The transformation method according to claim 7, further comprising the steps of:
inducing spore formation in a spore formation medium after selecting a strain in which the target gene has been introduced; and culturing asci isolated from the spore formation medium in a germination medium.

10. A method for producing a substance, comprising the step of culturing transformed yeast prepared by introducing multiple target genes into the yeast for transformation according to any one of claims 1 to 6.

11. The method for producing a substance according to claim 10, wherein the target gene is a β-glucosidase gene and an object to be produced is ethanol that is produced using an oligosaccharide as a substrate.

12. The method for producing a substance according to claim 10, wherein the target gene is a lactate dehydrogenase gene and an object to be produced is lactic acid.

13. The method for producing a substance according to claim 10, wherein the target gene is expressed under the control of a pyruvate decarboxylase gene (PDC1) promoter.

14. A method for providing multiple selection markers to yeast having homothallic properties, comprising the steps of:
introducing a DNA fragment to yeast having homothallic properties and being imparted auxotrophy by deficiency of an auxotrophy-related gene involved in synthesis of a predetermined nutritional component, wherein the DNA fragment comprises an upstream region of a deletion-target auxotrophy-related gene that is involved in synthesis of a nutritional component differing from the predetermined nutritional component, the auxotrophy-related gene, and a downstream region of the deletion-target auxotrophy-related gene in such order;
identifying a strain into which the DNA fragment has been introduced using the auxotrophy as an index;
selecting a strain to which auxotrophy has been imparted again after the loss of the above auxotrophy-related gene, from among strains identified in the above steps;
inducing spore formation by inoculating the strain selected in the above step in a spore formation medium; and
culturing asci isolated from the spore formation medium in a germination medium.

15. The method for providing multiple selection markers to yeast having homothallic properties according to claim 14, wherein the nutritional component is an amino acid or a base and the auxotrophy-related gene is an amino acid synthesis-related gene or a base synthesis-related gene.

16. The method for providing multiple selection markers to yeast having homothallic properties according to claim 14, wherein the nutritional component is tryptophan, histidine, or uracil.
